# EUROPÄISCHE PATENTSCHRIFT

(11) **EP 2 361 049 B1**
(45) Veröffentlichungstag und Bekanntmachung des Hinweises auf die Patenterteilung: **19.03.2014**
(21) Anmeldenummer: 09749053.6
(22) Anmeldetag: 28.10.2009
(51) Int. Cl.: A61B 19/00

(54) **MAGNETSPULENANORDNUNG MIT FESTEN UND BEWEGLICHEN SPULEN**
MAGNETIC COIL ASSEMBLY HAVING FIXED AND MOVABLE COILS
AGENCEMENT DE BOBINES MAGNÉTIQUES AVEC BOBINES FIXES ET MOBILES

(30) Priorität: 22.12.2008 DE 102008064379
(43) Veröffentlichungstag der Anmeldung: 31.08.2011
(73) Patentinhaber: Siemens Aktiengesellschaft, 80333 München (DE)
(72) Erfinder: REINSCHKE, Johannes, 90425 Nürnberg (DE); RÖSNER, Andreas, 91126 Rednitzhembach (DE)
(86) Internationale Anmeldenummer: PCT/EP2009/064186
(87) Internationale Veröffentlichungsnummer: WO 2010/072447

(56) Entgegenhaltungen:
- EP-A1- 2 036 483
- WO-A1-2007/054404
- WO-A2-2005/042053
- DE-A1-102006 060 421

## Beschreibung

Die Erfindung betrifft eine Magnetspulenanordnung mit mehreren Einzelspulen zur berührungslosen Führung eines magnetischen Elements, insbesondere einer magnetischen Endoskopiekapsel, in einem Arbeitsraum, wobei zumindest eine der Einzelspulen beweglich ist.

Die Verwendung von magnetischen, berührungslos navigierbaren Endoskopiekapseln zur Diagnose oder zur Behandlung des Inneren eines Patienten ist eine attraktive Alternative zur standardmäßigen Verwendung katheter- oder drahtgeführter Endoskope. Eine "berührungslose" Navigation im Sinne der Erfindung meint dabei, dass die Endoskopiekapsel katheter- oder drahtlos geführt werden kann. Ein Magnetspulensystem zur berührungslosen Navigation einer Endoskopiekapsel wird bspw. in der DE 10 2005 010 489 B4 beschrieben. Das Magnetspulensystem besteht dort aus 14 Einzelspulen zur Navigation einer Endoskopiekapsel, einer Videokapsel oder einer sonstigen Sonde. Die Kapsel ist hierbei mit einem magnetischen Element, bspw. mit einem Permanentmagneten, ausgestattet. Die Einzelspulen der Magnetspulenanordnung erzeugen Magnetfeldkomponenten Bx, By, Bz entlang der Achsen x, y, z eines kartesischen Koordinatensystems sowie magnetische Gradientenfelder oder ein Magnetfeldmaximum an einer vorgebbaren Position. Dies ermöglicht eine berührungslose Führung der Endoskopiekapsel: Die Felder wechselwirken mit dem magnetischen Element, wodurch magnetische Kräfte und Drehmomente generiert werden, welche die Bewegung des magnetischen Elements verursachen. Über eine entsprechende Bestromung in den einzelnen Spulen des Magnetspulensystems kann dabei die magnetische Kraft und das magnetische Drehmoment, welche auf das magnetische Element wirken, geeignet eingestellt werden. Die Bestromung der Einzelspulen des Magnetspulensystems hängt jedoch nicht nur von den aufzubringenden Kräften und Drehmomenten ab, sondern auch von der Position der Kapsel relativ zur Magnetspulenanordnung. Um die Kapsel navigieren zu können muss daher die momentane Kapselposition sehr genau bekannt sein. Aus diesem Grund sind bspw. in der DE 10 2005 010 489 B4 Mittel zur Detektion der Position vorgesehen.

Bei der mit den bekannten Magnetspulenanordnungen ausführbaren magnetisch geführten Kapselendoskopie ist es insbesondere beim Magen-Screening, wie es bspw. in WO 2007 / 077922 A1 beschrieben wird, erwünscht, dass die Endoskopiekapsel in einer vorgebbaren Kapselposition gehalten werden kann. Während der Magen-Screening-Prozedur ist der Magen teilweise mit Wasser gefüllt, und die Kapsel soll sich im Wasser oder an der Wasseroberfläche bewegen. Das magnetische Halten der Endoskopiekapsel an einer vorgebbaren Position ist besonders wichtig, wenn sich die Kapsel an der Wasseroberfläche befindet. Hierfür werden Magnetfelder generiert, deren maximale Stärke an der Soll-Position der Kapsel auftritt. Die Feldgradienten in der Nähe des Feldmaximums führen zu magnetischen Kräften auf die Kapsel, die auf die Kapsel eine Kraftwirkung in Richtung auf das Feldmaximum ausüben. Da die mechanischen Stör- und Gegenkräfte auf die Kapsel beim Magen-Screening sehr gering sind, positioniert sich die Kapsel letztendlich im Feldmaximum.

Die Endoskopiekapsel ist mit Hilfe der Magnetspulenanordnung in einem festen Arbeitsvolumen navigierbar. Die räumliche Ausdehnung des Arbeitsvolumens hängt von der Konfiguration der Magnetspulenanordnung ab. Dabei spielt sowohl die Geometrie der Magnetspulenanordnung eine Rolle, d.h. die Formen bzw. Querschnitte und die Anordnung der Einzelspulen der Magnetspulenanordnung sowie deren Maße, als auch bspw. die Auswahl der Leistungsverstärker zur Bestromung der Einzelspulen. Die Zahl der Leistungsverstärker wird nachstehend synonym zum Begriff "Anzahl der elektrischen Freiheitsgrade" verwendet.

Will man ein zunächst starr angenommenes Magnetspulensystem mit hinreichend vielen elektrischen Freiheitsgraden für die magnetische Kapselendoskopie, insbesondere für das Magen-Screening, auslegen, so macht man folgende Feststellung. Soll an einem vorgebenen Kapselort ein magnetisches Grundfeld sowie ein magnetische Gradientenfeld jeweils innerhalb vorgegebener Grenzen frei eingestellt werden können, so muss sich die Kapsel in einem ersten Arbeitsvolumen befinden. Möchte man am Kapselort ein magnetisches Grundfeld sowie ggf. ein magnetisches Gradientenfeld erzeugen und fordert darüber hinaus, dass das Magnetfeld am Kapselort ein (lokales) Maximum hat, dann muss sich die Kapsel in einem zweiten Arbeitsvolumen befinden. Dabei ist das zweite Arbeitsvolumen ein Teilvolumen des ersten Arbeitsvolumens, d.h. das zweite Arbeitsvolumen ist kleiner als das erste Arbeitsvolumen.

Während bei einem liegenden Patienten die Lage des Magens in vertikaler Richtung, im Folgenden als y-Richtung eines kartesischen Koordinatensystems bezeichnet, relativ gut bekannt ist, ist die Unsicherheit der Position des Magens in einer horizontalen Ebene, d.h. in x- und in z-Richtung des kartesischen Koordinatensystems, vergleichsweise groß. Wäre die horizontale Position des Magens genau bekannt, würde für ein Magen-Screening eine horizontale Ausdehnung des Arbeitsvolumens von etwa 20cm x 20cm ausreichen.

Typischerweise muss das Arbeitsvolumen jedoch einschließlich notwendiger Sicherheitsabstände und aufgrund der erwähnten Unbestimmtheit der Position des Magens mit etwa 30cm x 30cm x 30cm vergleichsweise groß gewählt werden. Mit den bisher bekannten Magnetspulensystemen und mit den zur Berechung der Bestromungsmuster der Einzelspulen verwendeten Algorithmen zur Erzeugung der gewünschten Magnetfelder etc. ist es jedoch nicht möglich, den Ort des Feldmaximums zur Positionierung der Endoskopiekapsel völlig frei in einem solch großen Arbeitsvolumen zu wählen. Vielmehr muss ein in x-, y- und z-Richtung mit etwa 20cm x 30cm x 20cm deutlich kleineres Teilvolumen definiert werden, in dem sich das Feldmaximum befinden muss. Abgesehen von der Notwendigkeit eines vergrößerten Arbeitsvolumens speziell für das Magen-Screening wäre es generell von Vorteil, über ein Magnetspulensystem zu verfügen, mit dem eine Endoskopiekapsel in einem möglichst großen Raumgebiet navigiert werden kann.

Die Druckschrift WO 2005/042053 A2 offenbart eine Magnetspulenanordnung gemäß dem Oberbegriff des Anspruchs 1. Ferner ist aus der EP 2 036 483 A1 ein System zum Führen eines medizinischen Geräts bekannt.

Die vorliegende Erfindung setzt sich daher zum Ziel, eine Magnetspulenanordnung zu schaffen, mit der eine Endoskopiekapsel in einem großen Arbeitsvolumen navigierbar ist.

Diese Aufgabe wird durch den Gegenstand des Anspruchs 1 gelöst. Vorteilhafte Ausgestaltungen ergeben sich aus den abhängigen Ansprüchen.

Bei der erfindungsgemäßen Lösung wird eine Magnetspulenanordnung zur berührungslosen Führung eines magnetischen Elements in einem Arbeitsraum mit einer Vielzahl von Einzelspulen vorgeschlagen, bei der neben raumfesten, unbeweglichen Einzelspulen auch bewegliche Einzelspulen vorgesehen sind. Dies hat den Vorteil, dass mit Hilfe der beweglichen Einzelspulen das Arbeitsvolumen im Raum verschoben werden kann, so dass effektiv ein größeres Arbeitsvolumen geschaffen wird.

Vorteilhafterweise ist zumindest eine weitere Einzelspule beweglich. Die diversen Magnet- und Gradientenfelder zur Navigation der Kapsel werden durch Spulenpaare jeweils bestehend aus zwei Einzelspulen erzeugt. Es ist daher von Vorteil, wenn zumindest zwei Einzelspulen, d.h. ein Spulenpaar, beweglich ist.

Die Einzelspulen sind mit einem raumfest installierten Halterahmen verbunden, wobei die unbeweglichen Einzelspulen fest mit dem Rahmen verbunden sind, während die bewegliche Einzelspule einen Führungswagen aufweist, der auf einer am Halterahmen fest installierten Führungsschiene gelagert ist. Durch einen derartigen Halterahmen wird vorteilhafterweise eine ausreichende Stabilität des Gesamtsystems erreicht. Darüber hinaus wird für die beweglichen und die unbeweglichen Einzelspulen ein gemeinsames Bezugssystem geschaffen.

In einer vorteilhaften Ausgestaltung sind zumindest zwei Spulenpaare beweglich. Ist nur ein Spulenpaar beweglich, so ist auch nur die eine durch dieses erste bewegliche Spulenpaar erzeugbare Magnetfeldkomponente bzw. das eine erzeugbare Gradientenfeld verschiebbar. D.h. dass in dem Teil des Arbeitsvolumens, das durch die Verschiebung des ersten Spulenpaares zum ursprünglichen Arbeitsvolumen hinzukommt, nur die durch das erste Spulenpaar erzeugbaren Felder verfügbar sind. Dies bedeutet, dass entsprechend wenige Freiheitsgrade zur Kapselnavigation zur Verfügung stehen. Dieser Nachteil wird durch die vorteilhafte Ausgestaltung mit mehreren beweglichen Spulenpaaren ausgeräumt. In einer noch weiter gehenden Ausgestaltung sind die beweglichen Spulenpaare gemeinsam beweglich, d.h. sie werden um denselben Betrag und in dieselbe Richtung verschoben.

In einer weiteren Ausgestaltung ist eine Patientenliege vorgesehen, auf der bspw. ein zu untersuchender Patient gelagert wird. Die Patientenliege ist derart in der Magnetspulenanordnung positionierbar, dass der zu untersuchende Körperbereich des Patienten, bspw. der Magen, in dem Arbeitsvolumen der Magnetspulenanordnung liegt. Erst hiermit wird die eigentliche Untersuchung des Patienten möglich.

Weitere Vorteile, Merkmale und Einzelheiten der Erfindung ergeben sich aus dem im Folgenden beschriebenen Ausführungsbeispiel sowie anhand der Zeichnungen.

Dabei zeigt:
- Figur 1: ein System für eine MGCE-Untersuchung,
- Figur 2: eine schematische Darstellung einer Magnetspulenanordnung,
- Figur 3: eine dreidimensionale Darstellung einer Magnetspulenanordnung mit verschiebbaren Spulen und
- Figur 4: ein Spulenpaar bestehend aus zwei Spulenarrays.

In den Figuren sind identische bzw. einander entsprechende Bereiche, Bauteile, Bauteilgruppen oder Verfahrensschritte mit denselben Bezugsziffern gekennzeichnet.

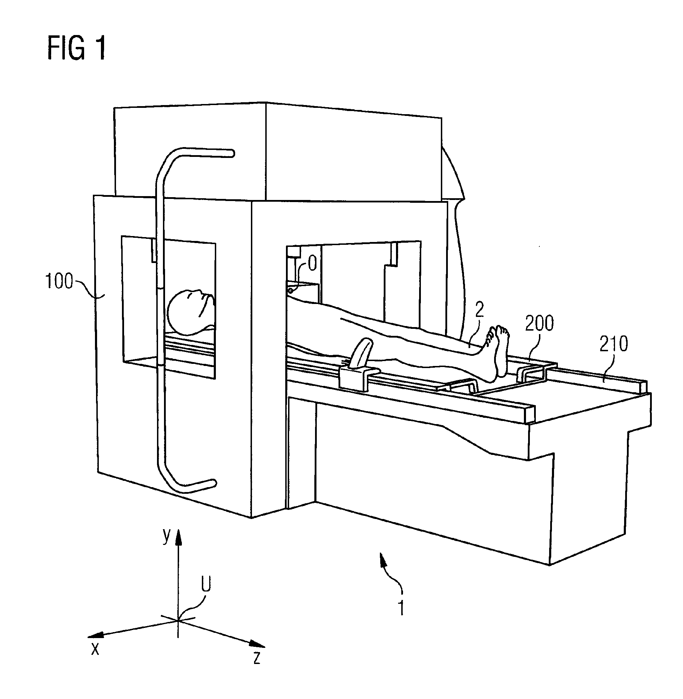

Die Figur 1 zeigt ein Übersichtsbild eines Systems 1, mit dem eine magnetisch geführte Kapselendoskopie durchgeführt werden kann. Das System 1 weist eine Magnetspulenanordnung 100 und eine Liege 200 auf, auf der ein Objekt zur Untersuchung gelagert wird. Ein Objekt ist dabei bspw. ein menschlicher, tierischer, technischer oder allgemein ein physikalischer Körper.

Speziell im Falle einer Magen-Screening-Untersuchung eines Patienten 2 handelt es sich bei der Liege 200 um eine Patientenliege, auf der der Patient 2 zur Untersuchung bzw. Behandlung gelagert wird. Die Patientenliege 200 befindet sich innerhalb einer Röhre der Magnetspulenanordnung 100 und ist zumindest in Richtung ihrer Längsachse, die die z-Achse eines kartesischen Koordinatensystems definiert, bspw. auf Schienen 210 verschiebbar. Die Längsachse der Patientenliege 200 ist dabei definitionsgemäß entlang der größten Ausdehnung der Patientenliege ausgerichtet. Zur Untersuchung wird die Patientenliege 200 zunächst soweit aus der Magnetspulenanordnung 100 herausgefahren, dass sich der Patient 2 bequem auf die Liege 200 legen kann. Anschließend wird die Patientenliege 200 mit dem Patienten 2 so weit in die Magnetspulenanordnung 100 gefahren, so dass ein Arbeitsvolumen der Magnetspulenanordnung 100, in dem eine Endoskopiekapsel mit Hilfe der Magnetspulenanordnung 100 navigiert werden kann und das im Folgenden näher erläutert wird, mit einem mit der Kapsel zu untersuchenden Raumbereich, bspw. mit dem Magen des Patienten 2, zusammenfällt. Mit anderen Worten ist dann die Patientenliege 200 derart im Bereich der Magnetspulenanordnung 100 angeordnet, dass das Arbeitsvolumen mit einem Raumbereich der Patientenliege 200 zusammenfällt, in dem sich ein mit der Magnetspulenanordnung 100 zu untersuchendes, auf der Liege 200 gelagertes Objekt befindet.

Die Vertikale entspricht wie bereits erwähnt der y-Richtung. Folglich ist die x-Achse des Koordinatensystems in der Horizontalebene senkrecht zur Längsachse der Patientenliege 200 und zur Längsachse der Röhre der Magnetspulenanordnung 100 orientiert. Der Ursprung 0 des kartesischen Koordinatensystems liegt raumfest, d.h. unbeweglich relativ zu einem nicht dargestellten Raum, in dem das System 1 installiert ist, im Zentrum der die Patientenliege 200 umgebenden Magnetspulenanordnung 100, wenn diese sich in einer Ausgangsposition befindet.

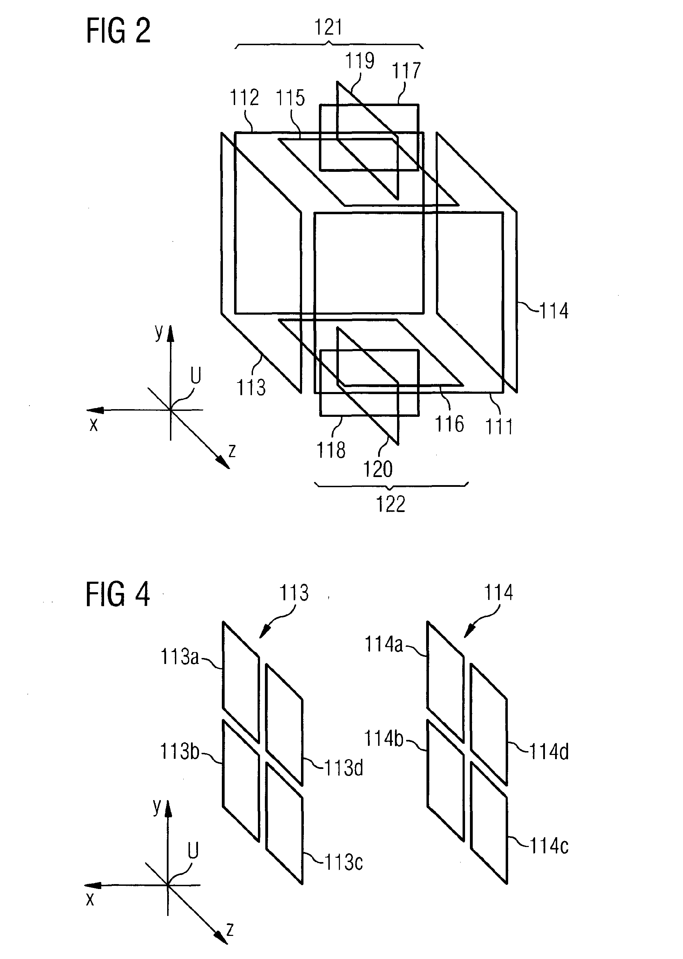

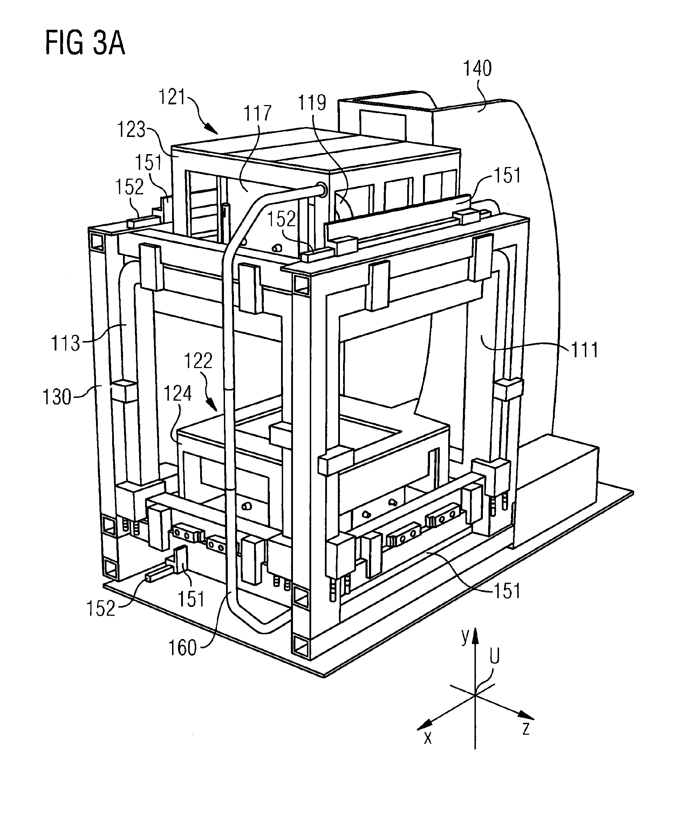

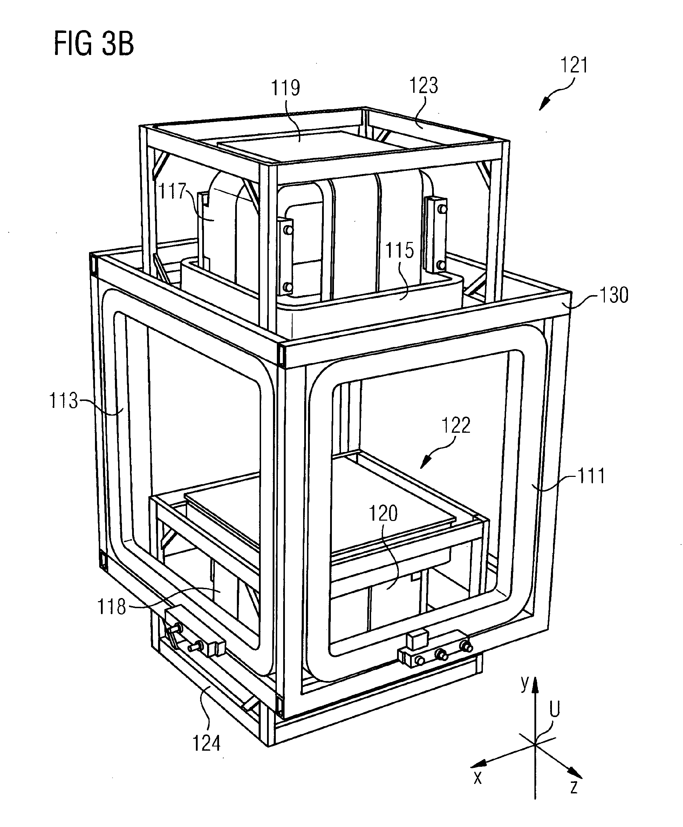

In der Figur 1 ist wie auch in den Figuren 2 und 3 jeweils ein Koordinatensystem x,y,z angedeutet. Der Ursprung U dieses Koordinatensystems x,y,z ist jedoch nicht mit dem Ursprung 0 zu verwechseln. Die Achsen der Koordinatensysteme mit den Ursprüngen U und 0 sind parallel.

Die Magnetspulenanordnung 100 umfasst, wie im Folgenden im Zusammenhang mit der Figur 2 beschrieben wird, eine Vielzahl von Einzelspulen 111-120, wobei erfindungsgemäß einige der Einzelspulen beweglich bzw. verschiebbar sind, während die übrigen Einzelspulen unbeweglich sind. Die Ausgangsposition der Magnetspulenanordnung 100 zeichnet sich dadurch aus, dass die beweglichen Einzelspulen nicht verschoben sind, sich also in einer Nullstellung befinden. In der Ausgangsposition weist die Magnetspulenanordnung 100 bspw. die Form eines Quaders oder Zylinders mit rechteckiger Grundfläche auf, d.h. eine symmetrische Form, während die Symmetrie bei verschobenen Einzelspulen verloren geht.

Die Magnetspulenanordnung 100 umfasst bspw. zehn Einzelspulen 111-120, die wie in der Figur 2 dargestellt angeordnet sein können, um die für ein Magen-Screening notwendigen Manöver der Endoskopiekapsel (nicht dargestellt) ausführen zu können. Eine derartige Magnetspulenanordnung 100 wird nebst den hiermit erzeugbaren Magnet- und Gradientenfeldern sowie den möglichen Kapselmanövern in der zum Zeitpunkt der Anmeldung der vorliegenden Erfindung noch nicht veröffentlichten Patentanmeldung DE 10 2008 004 871 beschrieben. Der Ursprung 0 des kartesischen Koordinatensystems befindet sich auch hier im Zentrum der Magnetspulenanordnung 100, wenn sich diese in der Ausgangsposition befindet.

Mit Hilfe der Magnetspulenanordnung 100 lässt sich eine Endoskopiekapsel in einem Arbeitsvolumen bewegen, das typischerweise 20 x 30 x 20cm³ groß ist und dessen Mittelpunkt mit dem Ursprung 0 des kartesischen Koordinatensystems zusammenfällt. Unter diesem Arbeitsvolumen, im Folgenden als technisches Arbeitsvolumen V_{tech} bezeichnet, wird dabei der Raumbereich verstanden, in dem die Kapsel mit der Magnetspulenanordnung 100 den Vorgaben bzgl. magnetischer Kraft und Drehmoment, Positioniergenauigkeit und Positionsstabilität (gegen kleine Störkräfte, z.B. aufgrund einer leichten Wasserbewegung) entsprechend bewegt werden kann.

Effektiv lässt sich ein gegenüber dem technischen Arbeitsvolumen V_{tech} vergrößertes Arbeitsvolumen V_{eff} schon dadurch erzeugen, da die Patientenliege 200 in z-Richtung verschiebbar ist. Unter der Annahme, dass die Patientenliege 200 in positive und negative z-Richtung jeweils um einen Betrag z₀ [cm] verschoben werden kann, ergibt sich ausgehend vom technischen Arbeitsvolumen V_{tech} = 20 x 30 x 20cm³ ein effektives Arbeitsvolumen V_{eff} von 20 x 30 x (20+2z₀)cm³. Dabei wird nicht das technische Arbeitsvolumen V_{tech} vergrößert, sondern das Arbeitsvolumen wird erfindungsgemäß im Raum bzgl. des Ursprungs 0 des Koordinatensystems verschoben, so dass sich ein effektives Arbeitsvolumen V_{eff} ergibt, das gegenüber dem technischen Arbeitsvolumen V_{tech} um einen Betrag vergrößert ist, der vom Betrag z₀ der Verschiebung abhängt.

Um ein effektives Arbeitsvolumen V_{eff} zu erzeugen, das auch in x-Richtung, d.h. in der horizontalen Richtung senkrecht zur Längsachse bzw. zur Verschiebungsrichtung der Patientenliege 200, gegenüber dem technischen Arbeitsvolumen V_{tech} vergrößert ist, werden einige der Einzelspulen 111-120 der Magnetspulenanordnung 100 verschiebbar angeordnet. Die entsprechenden Einzelspulen werden dazu an Führungswagen befestigt, die auf Führungsschienen gleiten oder rollen.

Die Figur 2 zeigt die Prinzipskizze einer Magnetspulenanordnung 100 bestehend aus 10 Einzelspulen 111-120. Die Einzelspulen 111-116 sind nach ihrer Bauart Racetrack-Spulen mit rechteckigem Querschnitt auf den Seitenflächen eines Quaders oder Zylinders mit rechteckiger Grundfläche angeordnet, während die Einzelspulen 117-120 an den Seiten des Quaders oder Zylinders "flügelartig" angeordnet bzw. eingesetzt sind. Dabei sagt der Begriff "flügelartig" aus, dass die Querschnittsfläche einer flügelartig angeordneten Einzelspule senkrecht zur nächstliegenden Seitenfläche des Quaders bzw. Zylinders steht. Bspw. liegt die Querschnittsfläche der flügelartig angeordneten Einzelspule 117 in der x-y-Ebene und steht gleichzeitig senkrecht auf der Seitenfläche des Quaders, die durch die Einzelspule 115 definiert wird und welche parallel zur x-z-Ebene liegt. Entsprechendes gilt für die Einzelspulen 118-120.

Die Einzelspulen 115, 117 und 119 sind ineinander verschachtelt angeordnet und bilden einen Blockmagneten 121. Entsprechendes gilt für die Einzelspulen 116, 118 und 120, die in einem Blockmagneten 122 zusammengefasst sind.

Die für ein Magen-Screening notwendigen Komponenten Bx, By, Bz eines Magnetfeldes sowie die Gradientenfelder wie bspw. dBx/dy werden durch die Einzelspulen 111-120 erzeugt. Dabei werden in der Regel jeweils zwei Einzelspulen zu einem Spulenpaar zusammengefasst. Bspw. bilden die Einzelspulen 117 und 118 ein Spulenpaar zur Erzeugung eines Gradientenfeldes dBz/dy. Entsprechend bilden die Einzelspulen 113 und 114 ein Spulenpaar zur Erzeugung der Magnetfeldkomponente Bx in x-Richtung. Die Einzelspulen werden zur Erzeugung der Magnetfelder mit Hilfe entsprechender Leistungsverstärker bestromt. Je nach Bestromung der Einzelspulen ist es bspw. bei dem aus den Einzelspulen 113 und 114 bestehenden Spulenpaar möglich, sowohl die Magnetfeldkomponente Bx als auch ein Gradientenfeld dBx/dx zu erzeugen. Ersteres wird erreicht, wenn die Einzelspulen 113 und 114 in gleicher Weise bestromt werden, d.h. durch die beiden Spulen fließt der gleiche Strom in der gleichen Umlaufrichtung. Hierbei ist es möglich, beide Spulen mit nur einem, gemeinsamen Verstärker zu versorgen. Werden die Spulen dagegen unterschiedlich bestromt, so erzeugen die Einzelspulen 113 und 114 unterschiedliche Magnetfelder Bx(113) und Bx(114). Dementsprechend wird ein Gradientenfeld dBx/dx aufgebaut. Hierzu sind jedoch im Allgemeinen zwei Verstärker notwendig.

Allgemein bilden diejenigen Einzelspulen ein Spulenpaar, die sich in der Ausgangsposition der Magnetspulenanordnung 100 bezüglich des geometrischen Mittelpunktes der Magnetspulenanordnung 100 gegenüber liegen und deren Querschnittsflächen zueinander weitestgehend parallel sind.

Die Figur 3A zeigt eine dreidimensionale Ansicht der Magnetspulenanordnung 100 des in der Figur 1 dargestellten Systems 1. Die Einzelspulen 111-120, von denen in der Figur 3 nur einige mit Bezugszeichen versehen sind, sind in der Ausgangsposition wie in der Figur 2 dargestellt angeordnet und werden in einem bzgl. des Koordinatensystems ortsfesten Halterahmen 130 befestigt. Die Blockmagnete 121, 122 weisen jeweils einen Rahmen 123, 124 auf, wobei die Einzelspulen 115, 117 und 119 am Rahmen 123 befestigt sind, während die Einzelspulen 116, 118 und 120 am Rahmen 124 befestigt sind. Sowohl die Einzelspulen 111-116 als auch die Blockmagnete 121, 122 bzw. deren Rahmen 123, 124 sind mit dem Halterahmen 130 verbunden.

Die Blockmagnete 121, 122 sind in y-Richtung gesehen übereinander angeordnet und über einen C-Bogen 140 miteinander mechanisch verbunden. Darüber hinaus sind die Rahmen 123, 124 der Blockmagnete 121, 122 jeweils mit Hilfe von Führungswagen 151 verschiebbar auf Führungsschienen 152 gelagert, wobei die Führungswagen 151 auf den Führungsschienen 152 rollen oder gleiten. Die Führungswagen 151 und Führungsschienen 152 sind in x-Richtung ausgerichtet, so dass die Blockmagnete 121, 122 gemeinsam in positive und negative x-Richtung um jeweils einen Betrag x₀ verschiebbar sind. Demzufolge sind die drei aus den Einzelspulen 115 und 116, 117 und 118 sowie 119 und 120 gebildeten Spulenpaare gemeinsam in der x-Richtung verschiebbar. Das effektive Arbeitsvolumen V_{eff} überdeckt damit einen Bereich von (20+2x₀) x 30 x (20+2z₀)cm³, wobei auch die Verschiebbarkeit der Patientenliege 200 in z-Richtung berücksichtigt wurde.

Zur manuellen Verschiebung der Blockmagnete 121, 122 ist ein Griff 160 vorgesehen. Der Griff 160 ist mit den Rahmen 123, 124 der Blockmagnete 121, 122 verbunden. Alternativ kann die Verschiebung der Blockmagnete 121, 122 auch mit Hilfe eines elektrischen, pneumatischen oder eines sonstigen Antriebes an Stelle des Griffes 160 realisiert werden (nicht dargestellt).

Die Figur 3B zeigt die Magnetspulenanordnung 100 in einer übersichtlicheren Darstellung, in der insbesondere der C-Bogen 140, der Griff 160 sowie die Führungswagen 151 und Führungsschienen 152 nicht dargestellt sind.

In einer alternativen, nicht separat dargestellten Ausführungsform sind die vier Einzelspulen 111-114 zusammen mit der Patientenliege 200 in der x-Richtung verschiebbar. Die Blockmagnete 121, 122 umfassend die Einzelspulen 115-120 sind dagegen starr ausgeführt, d.h. sie sind gegenüber dem Ursprung 0 des kartesischen Koordinatensystems nicht bewegbar. Dies kann bspw. dadurch realisiert werden, dass die Blockmagnete 121, 122 bzw. deren Rahmen 123, 124 fest mit dem Halterahmen 130 verbunden sind, während die Einzelspulen 111-114 fest miteinander und mit der Patientenliege 200 verbunden sind. Auf dem Sockel 220 der Patientenliege 200, der raumfest installiert ist und an dem auch der Halterahmen 130 befestigt ist, sind in diesem Fall Führungsschienen vorgesehen, auf denen Führungswagen verschiebbar gelagert sind. Führungswagen und Führungsschienen sind auch in diesem Ausführungsbeispiel in der x-Richtung orientiert. Die Führungswagen sind an einem Rahmen befestigt, an dem des weiteren die beweglichen Einzelspulen 111-114 und die Patientenliege 200 befestigt sind.

Folglich sind die Einzelspulen 111-114 bzw. die beiden aus den Einzelspulen 111 und 112 bzw. 113 und 114 gebildeten Spulenpaare und die Patientenliege 200 in der x-Richtung verschiebbar. Auch in dieser Ausführungsform ist das effektive Arbeitsvolumen in x-Richtung entsprechend der möglichen Verschiebung x0 gegenüber dem technischen Arbeitsvolumen V_{tech} vergrößert.

Um das effektive Arbeitsvolumen V_{eff} in z-Richtung gegenüber dem technischen Arbeitsvolumen V_{tech} zu vergrößern, kann an Stelle einer verschiebbaren Patientenliege auch vorgesehen sein, dass einige der Einzelspulen 111-120 bzw. einige der Spulenpaare der Magnetspulenanordnung 100 in z-Richtung verschiebbar sind.

Die Einzelspulen 113-116, die im in der Figur 2 dargestellten Ausführungsbeispiel einen Würfel definieren, können grundsätzlich auch wie in der DE 10 2008 004 871 als Sattelspulen ausgebildet sein und einen Kreiszylinder definieren. Die Einzelspulen 111 und 112 sind dann idealerweise als Spulen mit kreisrundem Querschnitt ausgebildet. Andere Geometrien wie bspw. in der Figur 8 der DE 10 2008 004 871 dargestellt sind natürlich ebenfalls denkbar. Auch die Querschnitte der Einzelspulen 111-120 sind nicht auf die oben beschriebenen, quadratischen Querschnitte beschränkt. Selbstverständlich eignen sich je nach Geometrie und Anforderung der Magnetspulenanordnung bspw. auch kreisrunde, elliptische, rechteckige oder sonstige Querschnittsformen.

Allgemein sind die Einzelspulen derart angeordnet, dass sie auf den Seitenflächen eines gedachten geometrischen Körpers liegen oder in die Seitenflächen eingesetzt sind. Der geometrische Körper kann bspw. ein Würfel oder ein Quader sein, der derart im kartesischen Koordinatensystem positioniert ist, dass sein geometrischer Mittelpunkt mit dem Ursprung 0 zusammenfällt und die mittig und senkrecht auf den Seitenflächen stehenden Normalenvektoren mit den Achsen des Koordinatensystems übereinstimmen. Alternativ kann der geometrische Körper ein Zylinder mit kreisringförmigem, elliptischem oder auch unregelmäßigem Querschnitt sein. Die Längsachse des Zylinders ist dann entlang der z-Richtung des Koordinatensystems ausgerichtet. Die Seitenflächen, auf denen die Einzelspulen angeordnet sind, wären dann zum Einen die Stirnseiten und zum Anderen die Mantelfläche des Zylinders.

Die Ausführungsbeispiele beschreiben eine Konfiguration, bei der die Blockmagnete 121, 122 gegenüber den übrigen Spulen 111-114 und gegenüber der Patientenliege 200 in x-Richtung verschiebbar sind, und eine Konfiguration, bei der die Blockmagnete 121, 122 raumfest fixiert sind und die übrigen Einzelspulen 111-114 gemeinsam mit der Patientenliege 200 ebenfalls in x-Richtung verschoben werden können. Je nach Bedarf sind natürlich auch Konfigurationen denkbar, bei denen bspw. einzelne Spulen oder Spulenpaare in der vertikalen Richtung verschoben werden können. Auch eine Konfiguration, bei der Verschiebungsmöglichkeiten in den verschiedenen Raumrichtungen kombiniert sind, bspw. eine Verschiebung eines Spulenpaares in der y-Richtung und die Verschiebung eines weiteren oder desselben Spulenpaares in der x-Richtung, ist möglich.

Der oben eingeführte Begriff "Spulenpaar" impliziert, dass nur zwei Einzelspulen einander zugeordnet sind. In der Figur 2 bilden bspw. die Einzelspulen 113 und 114 ein Spulenpaar. Es ist jedoch grundsätzlich denkbar, dass wie in der Figur 4 dargestellt die Einzelspulen 113 und 114 jeweils selbst aus mehreren Spulen zusammengesetzt sind (die übrigen Einzelspulen der Magnetspulenanordnung 100 wurden in der Figur 4 der Übersichtlichkeit wegen weggelassen). Bspw. können die Einzelspulen 113, 114 jeweils als sog. Spulenarray mit je vier Einzelspulen 113a-d und 114a-d realisiert sein. Ein Spulenpaar im Sinne der vorliegenden Erfindung würde in einem solchen Fall die Spulenarrays 113a-d und 114a-d umfassen, sich also aus acht Einzelspulen zusammensetzen. Spulenarrays aus mehr oder weniger als vier Spulen sind natürlich ebenfalls denkbar. Der Begriff des Spulenpaars umfasst all diese Ausführungsformen der Einzelspulen.

Bei der Verschiebung eines Spulenpaares ändert sich die Position der von der Magnetspulenanordnung 100 navigierten Endoskopiekapsel relativ zu dem bewegten Spulenpaar. Um eine präzise Navigation zu gewährleisten ist es wie anfangs erwähnt notwendig, die Kapselposition sehr genau zu kennen. Umgekehrt ändert sich, wenn die Relativposition der Kapsel variiert, die für ein bestimmtes Kapselmanöver, bspw. ein Stillhalten der Kapsel in einer bestimmten Position und Orientierung, notwendige Bestromung der betreffenden Einzelspulen bzw. Spulenpaare. Um dies zu gewährleisten, weist das System 1 eine nicht dargestellte Steuerungseinrichtung zur Ansteuerung der Einzelspulen der Magnetspulenanordnung 100 auf. Die Steuerungseinrichtung steuert oder regelt die Leistungsverstärker (ebenfalls nicht dargestellt) zur Bestromung der Einzelspulen. Die Steuerung läuft dabei so ab, dass die mechanische Bewegung von Spulen oder Spulenpaaren und die Änderung der Bestromung der Spulen oder Spulenpaare simultan erfolgen. Bspw. mit Hilfe von Wegsensoren, die an den im Zusammenhang mit der Figur 3 beschriebenen Führungswagen 151 oder Führungsschienen 152 angebracht sein können, kann eine Verschiebung von Spulen oder Spulenpaaren ermittelt werden. Die Sensoren sind mit der Steuerungseinrichtung verbunden, so dass in der Steuerungseinrichtung in Abhängigkeit von der ermittelten Verschiebung die der neuen Relativposition der Endoskopiekapsel zur Magnetspulenanordnung entsprechende Bestromung der Spulen der Magnetspulenanordnung 100 berechnet werden kann.

## Patentansprüche

1. Magnetspulenanordnung (100) zur berührungslosen Führung eines magnetischen Elements in einem Arbeitsvolumen in einem raumfesten kartesischen Koordinatensystem mit einem Ursprung (0), umfassend mehrere Einzelspulen (111-120), wobei
- zumindest eine der Einzelspulen relativ zum Ursprung (0) beweglich ist und
- zumindest eine weitere der Einzelspulen relativ zum Ursprung (0) unbeweglich ist, wobei die Einzelspulen mit einem raumfesten Halterahmen (130) verbunden sind und die zumindest eine unbewegliche Einzelspule fest mit dem Halterahmen (130) verbunden ist,
**dadurch gekennzeichnet, dass**
- die zumindest eine bewegliche Einzelspule mit einem Führungswagen (151) verbunden ist, der an einer am Halterahmen (130) befestigten Führungsschiene (152) gelagert ist.

2. Magnetspulenanordnung (100) nach Anspruch 1, **dadurch gekennzeichnet, dass** die bewegliche Einzelspule in einer ersten horizontalen Richtung (x) und/oder in einer zweiten horizontalen Richtung (z) und/oder in der vertikalen (y) Richtung beweglich ist.

3. Magnetspulenanordnung (100) nach einem der vorhergehenden Ansprüche, **dadurch gekennzeichnet, dass** eine weitere Einzelspule relativ zum Ursprung (0) beweglich ist, wobei die weitere bewegliche Einzelspule in einer ersten horizontalen Richtung (x) und/oder in einer zweiten horizontalen Richtung (z) und/oder in der vertikalen (y) Richtung beweglich ist.

4. Magnetspulenanordnung (100) nach Anspruch 3, **dadurch gekennzeichnet, dass** mehrere bewegliche Einzelspulen gemeinsam beweglich sind.

5. Magnetspulenanordnung nach einem der vorhergehenden Ansprüche, **dadurch gekennzeichnet, dass** ein Griff zum manuellen Bewegen der beweglichen Einzelspulen vorgesehen ist.

6. Magnetspulenanordnung nach einem der vorhergehenden Ansprüche, **dadurch gekennzeichnet**, die beweglichen Einzelspulen über Spindeln manuell oder elektrisch bewegbar sind.

7. Magnetspulenanordnung (100) nach einem der vorhergehenden Ansprüche, **dadurch gekennzeichnet, dass** jeweils zwei Einzelspulen, ein Spulenpaar bildend, einander zugeordnet sind, wobei zumindest ein Spulenpaar gegenüber dem Ursprung (0) beweglich ist.

8. Magnetspulenanordnung (100) nach Anspruch 7, **dadurch gekennzeichnet, dass** diejenigen beiden Einzelspulen ein Spulenpaar bilden,
- die sich in einer Ausgangsposition der Magnetspulenanordnung (100) bezüglich des geometrischen Mittelpunktes der Magnetspulenanordnung (100) gegenüber liegen und
- deren Querschnittsflächen zueinander weitestgehend parallel sind.

9. Magnetspulenanordnung (100) nach Anspruch 7 oder 8, **dadurch gekennzeichnet, dass** ein bewegliches Spulenpaar in einer ersten horizontalen Richtung (x) und/oder in einer zweiten horizontalen Richtung (z) und/oder in der vertikalen (y) Richtung beweglich ist.

10. Magnetspulenanordnung (100) nach einem der Ansprüche 7 bis 9, **dadurch gekennzeichnet, dass** zumindest ein weiteres Spulenpaar gegenüber dem Ursprung (0) beweglich ist.

11. Magnetspulenanordnung (100) nach Anspruch 10, **dadurch gekennzeichnet, dass** mehrere bewegliche Spulenpaare gemeinsam bewegbar sind.

12. Magnetspulenanordnung (100) nach einem der vorhergehenden Ansprüche, **dadurch gekennzeichnet, dass** eine Vielzahl von Einzelspulen auf Seitenflächen eines gedachten geometrischen Körpers angeordnet sind, wobei der geometrische Körper
- ein Zylinder mit kreisringförmigem, elliptischem oder unregelmäßigem Querschnitt ist, dessen Längsachse entlang der z-Richtung des kartesischen Koordinatensystems ausgerichtet ist, wobei die Seitenflächen die Mantelfläche und die Stirnseiten des Zylinders sind, oder
- ein Würfel oder ein Quader ist, wobei die Normalenvektoren der Seitenflächen des Würfels oder des Quaders mit den Achsen des kartesischen Koordinatensystems zusammenfallen.

13. Magnetspulenanordnung (100) nach Anspruch 12, **dadurch gekennzeichnet, dass** weitere Einzelspulen flügelartig angeordnet sind.

14. Magnetspulenanordnung (100) nach einem der vorhergehenden Ansprüche, **dadurch gekennzeichnet, dass** eine Liege (200) vorgesehen ist, deren Längsachse mit der z-Achse des kartesischen Koordinatensystems zusammenfällt und deren Liegefläche in der x-z-Ebene liegt, wobei die Liege (200) derart im Bereich der Magnetspulenanordnung (100) angeordnet ist, dass das Arbeitsvolumen mit einem Volumen im Bereich der Liege (200) zusammenfällt, in dem sich ein mit der Magnetspulenanordnung zu untersuchendes, auf der Liege (200) gelagertes Objekt befindet.

15. Magnetspulenanordnung nach Anspruch 14, **dadurch gekennzeichnet, dass** die Liege (200) gemeinsam mit der zumindest einen beweglichen Einzelspule bewegbar ist.

16. Magnetspulenanordnung nach Anspruch 14 oder 15, **dadurch gekennzeichnet, dass** die Liege (200) in z-Richtung verschiebbar ist.

17. Magnetspulenanordnung (100) nach einem der vorhergehenden Ansprüche, **dadurch gekennzeichnet, dass** die Einzelspulen (111-120) ausgebildet sind, um zumindest im technischen Arbeitsvolumen die Komponenten eines Magnetfeldes und/oder Gradientenfelder zur Führung des magnetischen Elementes zu erzeugen.

18. Magnetspulenanordnung (100) nach einem der vorhergehenden Ansprüche, **dadurch gekennzeichnet, dass** das magnetische Element eine Endoskopiekapsel ist.

## Claims

1. Magnetic coil arrangement (100) for contactless guidance of a magnetic element in a work volume in a stationary Cartesian coordinate system with an origin (0), comprising a plurality of individual coils (111-120), wherein
- at least one of the individual coils is mobile relative to the origin (0) and
- at least one further one of the individual coils is stationary relative to the origin (0), wherein the individual coils are connected to a stationary holding frame (130) and the at least one stationary individual coil is securely connected to the holding frame (130),
**characterized in that**
- the at least one mobile individual coil is connected to a guide carriage (151), which is mounted on a guide rail (152) attached to the holding frame (130).

2. Magnetic coil arrangement (100) according to Claim 1, **characterized in that** the mobile individual coil is mobile in a first horizontal direction (x) and/or in a second horizontal direction (z) and/or in the vertical (y) direction.

3. Magnetic coil arrangement (100) according to one of the preceding claims, **characterized in that** a further individual coil is mobile relative to the origin (0), the further mobile individual coil being mobile in a first horizontal direction (x) and/or in a second horizontal direction (z) and/or in the vertical (y) direction.

4. Magnetic coil arrangement (100) according to Claim 3, **characterized in that** a plurality of mobile individual coils can be moved together.

5. Magnetic coil arrangement according to one of the preceding claims, **characterized in that** provision is made for a grip for manual movement of the mobile individual coils.

6. Magnetic coil arrangement according to one of the preceding claims, **characterized in that** the mobile individual coils can be moved manually or electrically by means of spindles.

7. Magnetic coil arrangement (100) according to one of the preceding claims, **characterized in that** two individual coils, forming a coil pair, are in each case assigned to one another, with at least one coil pair being mobile in relation to the origin (0).

8. Magnetic coil arrangement (100) according to Claim 7, **characterized in that** it is those two individual coils that form a coil pair
- which, in an initial position of the magnetic coil arrangement (100), lie opposite one another in relation to the geometric centre of the magnetic coil arrangement (100) and
- which have cross-sectional areas that are largely parallel to one another.

9. Magnetic coil arrangement (100) according to Claim 7 or 8, **characterized in that** a mobile coil pair is mobile in a first horizontal direction (x) and/or in a second horizontal direction (z) and/or in the vertical (y) direction.

10. Magnetic coil arrangement (100) according to one of Claims 7 to 9, **characterized in that** at least one further coil pair is mobile in relation to the origin (0).

11. Magnetic coil arrangement (100) according to Claim 10, **characterized in that** a plurality of mobile coil pairs can be moved together.

12. Magnetic coil arrangement (100) according to one of the preceding claims, **characterized in that** a multiplicity of individual coils are arranged on the side faces of an imagined geometric body, wherein the geometric body
- is a cylinder with an annular, elliptical or irregular cross section, the longitudinal axis of which is aligned along the z-direction of the Cartesian coordinate system, wherein the side faces are the lateral face and the end sides of the cylinder, or
- a cube or a cuboid, wherein the normal vectors of the side faces of the cube or of the cuboid coincide with the axes of the Cartesian coordinate system.

13. Magnetic coil arrangement (100) according to Claim 12, **characterized in that** further individual coils are arranged in an aliform manner.

14. Magnetic coil arrangement (100) according to one of the preceding claims, **characterized in that** a couch (200) is provided, the longitudinal axis of which coincides with the z-axis of the Cartesian coordinate system and the bearing surface of which lies in the x/z-plane, wherein the couch (200) is arranged in the region of the magnetic coil arrangement (100) in such a way that the work volume coincides with a volume in the region of the couch (200) in which an object to be examined by the magnetic coil arrangement, mounted on the couch (200) is situated.

15. Magnetic coil arrangement according to Claim 14, **characterized in that** the couch (200) can be moved together with the at least one mobile individual coil.

16. Magnetic coil arrangement according to Claim 14 or 15, **characterized in that** the couch (200) can be displaced in the z-direction.

17. Magnetic coil arrangement (100) according to one of the preceding claims, **characterized in that** the individual coils (111-120) are configured to generate the components of a magnetic field and/or of gradient fields for guiding the magnetic element, at least in the technical work volume.

18. Magnetic coil arrangement (100) according to one of the preceding claims, **characterized in that** the magnetic element is an endoscopy capsule.

## Revendications

1. Agencement de bobines magnétiques (100) destiné au guidage sans contact d'un élément magnétique dans un volume de travail situé dans un système de coordonnées cartésiennes fixe dans l'espace ayant une origine (0), comprenant plusieurs bobines individuelles (111-120), dans lequel
- au moins une desdites bobines individuelles est mobile par rapport à l'origine (0) et
- au moins une autre desdites bobines individuelles est immobile par rapport à l'origine (0),
lesdites bobines individuelles étant reliées à un châssis-support (130) fixe dans l'espace et ladite au moins une bobine individuelle immobile étant solidaire du châssis-support (130),
**caractérisé en ce que** ladite au moins une bobine individuelle immobile est reliée à un chariot de guidage (151) qui est monté sur un rail de guidage (152) fixé sur le châssis-support (130).

2. Agencement de bobines magnétiques (100) selon la revendication 1, **caractérisé en ce que** la bobine individuelle mobile peut se déplacer selon une première direction horizontale (x) et/ou selon une deuxième direction horizontale (z) et/ou dans la direction verticale (y).

3. Agencement de bobines magnétiques (100) selon l'une des revendications précédentes, **caractérisé en ce qu'**une autre bobine individuelle est mobile par rapport à l'origine (0), ladite autre bobine individuelle mobile pouvant se déplacer selon une première direction horizontale (x) et/ou selon une deuxième direction horizontale (z) et/ou selon la direction verticale (y).

4. Agencement de bobines magnétiques (100) selon la revendication 3, **caractérisé en ce que** plusieurs bobines individuelles mobiles peuvent se déplacer ensemble.

5. Agencement de bobines magnétiques selon l'une des revendications précédentes, **caractérisé en ce qu'**il est prévu une poignée pour le déplacement manuel des bobines individuelles mobiles.

6. Agencement de bobines magnétiques selon l'une des revendications précédentes, **caractérisé en ce que** les bobines individuelles mobiles peuvent être déplacées manuellement ou électriquement par l'intermédiaire de broches.

7. Agencement de bobines magnétiques (100) selon l'une des revendications précédentes, **caractérisé en ce que** chaque fois deux bobines individuelles, formant une paire de bobines, sont associées l'une à l'autre, au moins une paire de bobines étant mobile par rapport à l'origine (0).

8. Agencement de bobines magnétiques (100) selon la revendication 7, **caractérisé en ce que** les deux bobines individuelles qui forment une paire de bobines sont celles
- qui sont en regard l'une de l'autre dans une position de départ de l'agencement de bobines magnétiques (100) par rapport au centre géométrique de l'agencement de bobines magnétiques (100) et
- dont les aires de section sont sensiblement parallèles l'une à l'autre.

9. Agencement de bobines magnétiques (100) selon la revendication 7 ou 8, **caractérisé en ce qu'**une paire de bobines mobiles peut se déplacer selon une première direction horizontale (x) et/ou selon une deuxième direction horizontale (z) et/ou selon la direction verticale (y).

10. Agencement de bobines magnétiques (100) selon l'une des revendications 7 à 9, **caractérisé en ce qu'**au moins une autre paire de bobines est mobile par rapport à l'origine (0).

11. Agencement de bobines magnétiques (100) selon la revendication 10, **caractérisé en ce que** plusieurs paires de bobines mobiles peuvent être déplacées ensemble.

12. Agencement de bobines magnétiques (100) selon l'une des revendications précédentes, **caractérisé en ce qu'**une pluralité de bobines individuelles sont disposées sur des faces latérales d'un corps géométrique imaginaire, ledit corps géométrique étant
- un cylindre de section transversale circulaire, elliptique ou irrégulière, dont l'axe longitudinal est orienté selon la direction z du système de coordonnées cartésiennes, les faces latérales étant la surface d'enveloppe et les faces d'extrémité du cylindre, ou
- un cube ou un parallélépipède où les vecteurs normaux des faces latérales du cube ou du parallélépipède coïncident avec les axes du système de coordonnées cartésiennes.

13. Agencement de bobines magnétiques (100) selon la revendication 12, **caractérisé en ce que** d'autres bobines individuelles sont disposées en forme d'aile.

14. Agencement de bobines magnétiques (100) selon l'une des revendications précédentes, **caractérisé en ce qu'**il est prévu une table (200), dont l'axe longitudinal coïncide avec l'axe z du système de coordonnées cartésiennes et dont la surface de couchage est située dans le plan x-z, ladite table (200) étant disposée dans la région de l'agencement de bobines magnétiques (100), de telle sorte que le volume de travail coïncide avec un volume situé dans la région de la table (200) et dans lequel se trouve un objet couché sur la table (200), qui doit être examiné avec l'agencement de bobines magnétiques.

15. Agencement de bobines magnétiques selon la revendication 14, **caractérisé en ce que** la table (200) peut être déplacée ensemble avec ladite au moins une bobine individuelle mobile.

16. Agencement de bobines magnétiques selon la revendication 14 ou 15, **caractérisé en ce que** la table (200) peut coulisser selon la direction z.

17. Agencement de bobines magnétiques (100) selon l'une des revendications précédentes, **caractérisé en ce que** les bobines individuelles (111-120) sont conçues pour produire, au moins dans le volume de travail technique, les composantes d'un champ magnétique et/ou des champs à gradient pour le guidage de l'élément magnétique.

18. Agencement de bobines magnétiques (100) selon l'une des revendications précédentes, **caractérisé en ce que** l'élément magnétique est une capsule endoscopique.
